# EUROPÄISCHE PATENTSCHRIFT

(11) **EP 0 234 405 B1**
(45) Veröffentlichungstag und Bekanntmachung des Hinweises auf die Patenterteilung: **21.12.1994**
(21) Anmeldenummer: 87101849.5
(22) Anmeldetag: 10.02.1987
(51) Int. Cl.: A61K 39/42

(54) **Verwendung eines immunglobulinhaltigen Präparates zur Prophylaxe und Therapie von AIDS beim Menschen**
Use of an immunoglobulin-containing composition in the prophylaxis and therapy of AIDS of human
application d'une composition contenant de l'immunoglobuline à la prophylaxie et au traitement thérapeutique du SIDA chéz l'homme

(30) Priorität: 17.02.1986 DE 3604947
(43) Veröffentlichungstag der Anmeldung: 02.09.1987
(73) Patentinhaber: Biotest Pharma GmbH, D-60528 Frankfurt (DE)
(72) Erfinder: Schleussner, Hans, Dipl.Chem.Dr., D-6000 Frankfurt am Main 70 (DE); Dichtelmüller, Herbert, Dr., D-6231 Sulzbach/Ts. (DE); Stephan, Wolfgang, Dipl.Chem.Dr., D-6073 Dreieich (DE); Lissner, Reinhard, Dipl.Chem.Dr., D-8761 Weilbach (DE)
(74) Vertreter: Beil, Hans Chr., Dr.

(56) Entgegenhaltungen:
- EP-A- 0 260 691
- BIOTECHNOLOGY, Band 2, Nr.8, August 1984; T.WILSON, Seiten 682-683#
- CHEMICAL ABSTRACTS, Band 103, Nr.17, 28 October 1985, Columbus, OH (US); A.M. PRINCE, Seite 413, Nr. 138446b#
- NATURE, Band 316, Nr.6023, 04 Juli 1985, London (GB); M.R.GUROFF et al., Seiten 72-74#
- THE LANCET, 26. September 1985; Seiten 695-697 (bzw. Seiten 1-5 des Nachdrucks)#
- THE ECONOMIST, 16. November 1985; Seite 89#
- NATURE, Band 316, 1985; GROOPMANN, Seite 12#

## Beschreibung

Die Erfindung betrifft die Verwendung eines aus potentiell infektösem, anti-HTLV/LAV-positivem Blut, Plasma, Serum oder Derivaten gewonnenen immunglobulinhaltigen Präparates zur Herstellung eines Arzneimittels zur therapeutischen Behandlung und zur Prophylaxe von AIDS beim Menschen.

Die erworbene Immunschwäche AIDS (acquired immunodeficiency syndrome) wird bekanntlich durch das Virus HTLV-III bzw. LAV hervorgerufen. Als Merkmal für die Auseinandersetzung des Immunsystems mit einem eingedrungenen HTLV-III/LAV-Partikel gilt die Ausbildung von Antikörper-Aktivitäten gegen HTLV-III/LAV-spezifische Proteine oder Glycoproteine. Durch entsprechende Antikörpertests ist es möglich geworden, z.B. seropositive Blutspender zu erkennen und diese von der Produktion von Blutprodukten auszuschließen.

Antikörper gegen HTLV-III sind jedoch nicht nur als diagnostisches Merkmal interessant. Wie bereits von zahlreichen anderen Viren bekannt ist, stellen körpereigene oder passiv zugeführte Antikörper z.B. gegen Hepatitis-B-Virus, Hepatitis-A-Virus, Cytomegalievirus, Varizella-zoster-Virus oder Influenzavirus einen wesentlichen Schutz vor Infektionen dar. Die prophylaktische Gabe oder postexpositionelle Prophylaxe mittels Immunglobulin oder Hyperimmunglobulinen mit entsprechender Antikörper-Aktivität ist in der Medizin in vielen Bereichen zu einem Standardverfahren von wesentlicher Bedeutung geworden.

Auch für den Erreger von AIDS, HTLV-III/LAV, sind neutralisierende Antikörper bekannt. Diese Antikörper treten in der frühen Phase nach Infektion auf, während der antikörperpositive Personen noch nicht unter Krankheitssymptomen leiden. Die späten Stadien der Erkrankung mit den bekannten Symptomen fallen mit einer Abnahme des Anti-HTLV-III-Titers zusammen.

Es erscheint somit zweckmäßig, Antikörpertiter, speziell von neutralisierenden Antikörpern, gegen HTLV-III in seropositiven Personen auf hohem Niveau zu halten, um eine Ausbreitung der Viren im Organismus zu verhindern.

Es ist deshalb wünschenswert, auch zur Prophylaxe oder Therapie einer Infektion und Erkrankung durch HTLV-III/LAV solche immunglobulinhaltigen oder Hyperimmunglobulin-Präparate bereitzustellen, zumal u.a. wegen der Antigenvariabilität ein Impfstoff gegen HTLV-III oder LAV derzeit nicht verfügbar ist und in der nächsten Zeit auch nicht verfügbar sein wird.

Aus Cancer Research (Suppl.) 45 (September 1985), S. 4592s bis 4594s sind Versuche zur quantitativen Bestimmung der Infektiosität von Blutpräparaten hinsichtlich HTLV-III und zur Sterilisation derartiger Präparate bekannt.

Aus A. Karpas, THE LANCET, 26.09.85, S. 695-697, und THE ECONOMIST, 16.11.85, S. 89, ist der Vorschlag bekannt, terminal kranke AIDS-Patienten mit Plasmatransfusionen zu behandeln. Die Plasmen sollen aus HIV-positiven, sonst aber gesunden Patienten gewonnen und nach den Kriterien eines hohen Titers an HIV-neutralisierenden Antikörpern unter Einsatz eines speziellen Screening-Verfahrens ausgewählt werden. Groopman berichtet in Nature, Vol. 316, 1985, S. 12, über die beabsichtigte Erprobung von sogenannten Hyperimmunglobulinen und Pools mit hohem Titer an HIV-neutralisierenden Immunglobulinen an Primaten.

Bei den bisherigen Verfahren zur Herstellung von z.B. Hyperimmunglobulin-Präparaten gegen bestimmte Viren, wie Hepatitis-B, wurde als Ausgangsmaterial Blut bzw. Plasma von solchen Spendern verwendet, die immunisiert waren bzw. eine Erkrankung bereits überstanden hatten und als gesund galten. Derartige Spender stehen jedoch im Fall von AIDS nicht zur Verfügung.

Gegenstand der vorliegenden Erfindung ist daher die Verwendung eines immunglobulinhaltigen Präparates, erhalten durch Aufarbeiten von potentiell infektösem, anti-HTLV-III/LAV-positivem Blut, Plasma, Serum oder deren Derivaten auf eine immunglobulinhaltige Lösung, Sterilisation oder gegebenenfalls Lyophilisation, zur Herstellung eines Arzneimittels zur therapeutischen Behandlung und Prophylaxe von AIDS beim Menschen.

Diese Aufgabe wird erfindungsgemäß dadurch gelöst, daß man als Ausgangsmaterial ein potentiell infektiöses, anti-HTLV-III/LAV-positives Blut, Plasma oder Serum oder Derivate derselben verwendet.

Für die erfindungsgemäße Verwendung werden Plasma- bzw. Blutspender ausgewählt, welche aufgrund ihres anti-HTLV-III-positiven Antikörperstatus als Überträger der Erkrankung gelten und normalerweise von der Blut-/Plasmaspende ausgeschlossen werden. Dies steht im überraschenden Gegensatz zu den bisher bekannten und angewandten Methoden zur Herstellung von Hyperimmunglobulin-Präparaten, bei denen potentiell infektiöse Blut- oder Plasmaspenden von der Weiterverarbeitung ausgeschlossen sind.

Zur Herstellung des erfindungsgemäß zu verwendenden Präparates wird zunächst ein Screening des Ausgangsmaterials auf Antikörper gegen HTLV-III/LAV durchgeführt, wobei als Antigen ganze Partikel des Virus oder HTLV-III/LAV-spezifische Proteine oder Glycoproteine eingesetzt werden. Entsprechende Testsysteme sind bekannt und im Handel erhältlich. Als Beispiele seien der Enzymimmunoassay (ELISA) von Abbott (Bayer et al, Lancet 1984 ii, 1347) und der Immunfluoreszenztest genannt. Nachdem durch das Screening mit Hilfe eines derartigen Testsystems anti-HTLV-III/LAV-positives Blut, Plasma oder Serum ausgewählt wurde, wird dieses gepoolt und auf eine immunglobulinhaltige Lösung aufgearbeitet. Die Aufarbeitung erfolgt in an sich bekannter Weise. Beispielsweise kann Serum oder Plasma durch Entfernen der labilen Proteine (Lipoproteine und Gerinnungsfaktoren), durch Aufbewahren bei tiefen Temperaturen, z.B. bei -80°C, oder durch Lyophilisieren in eine lagerstabile Form gebracht werden.

Gemäß einer bevorzugten Ausführungsform werden aus dem ausgewählten gepoolten Ausgangsmaterial, insbesondere dem Plasma oder Serum, die Immunglobuline, insbesondere die Immunglobuline der Klassen IgG und/oder IgM und/oder IgA, nach bekannten Verfahren isoliert. Zu den bekannten Isolierungsverfahren für die Immunglobuline gehören das Cohn-Verfahren, das auf der Fällung der verschiedenen Plasmafraktionen durch Alkohol beruht, die Fällung mittels Rivanol und/oder Ammoniumsulfat oder mittels Polyethylenglykol oder auch mit einer Kombination von Polyethylenglykol und Hydroxyethylstärke oder die Isolierung mittels Gelchromatographie, Säulenchromatographie an Ionenaustauschern, Elektrophorese oder Immunadsorption an im Handel erhältlichen Gelen. Bevorzugte Isolierungsverfahren für die Immunglobuline sind das Cohn-Verfahren, beispielsweise nach einer Variation von Kistler und Nitschmann (Vox Sang. 1962; 7, 414 bis 424), und ein kombiniertes Rivanol-Ammoniumsulfat-Trennungsverfahren (Ehrhart/Ruschig, Arzneimitel, 2. Auflage 1972, Bd. 5, S. 400).

Ein wesentliches Merkmal des erfindungsgemäß zu verwendenden Präparates ist, daß gegebenenfalls vor oder nach der Isolierung der Immunglobuline eine Sterilisation in an sich bekannter Weise durchgeführt werden kann. Eine sichere Sterilisation der erfindungsgemäß zu verwendenden Präparate ist eine unabdingbare Voraussetzung, um den therapeutischen Effekt nutzbar zu machen und zugleich eine Übertragung der Krankheit sicher auszuschließen. Zu den brauchbaren Sterilisationsverfahren gehören die Behandlung mit β-Propiolacton (z.B. in einer Konzentration von 0,1 bis 0,4 %), mit UV-Strahlen, Röntgen- oder Gammastrahlen, die Hitzebehandlung, die chemische Sterilisation (z.B. mittels Alkohol oder Formaldehyd) oder die Adsorption (z.B. an kolloidaler Kieselsäure wie Aerosil^{(R)} oder Kieselgur), die Behandlung bei niedrigen oder hohen pH-werten oder die Behandlung mit Detergentien (z.B. Sorbimacrogololeat, Natriumdodecylsulfat oder Tri-(n-butyl)-phosphat) oder Lösungsmitteln (z.B. Ether, Alkohol). Vorzugsweise wird die Sterilisation durch Behandlung mit β-Propiolacton und/oder UV-Strahlen durchgeführt.

Beispielsweise kann ein nach Entfernung des Kryopräzipitats und der Gerinnungsfaktoren II, VII, X und IX (PPSB-Faktoren) aus dem ausgewählten, gepoolten Plasma gemäß dem Cohn-Verfahren aufgearbeitetes und aus der Cohnfraktion II erhaltenes Immunglobulin-Präparat anschließend mit β-Propiolacton (0,14 %) sterilisiert werden.

Die Cohnfraktion III kann beispielsweise mit Octansäure und Calciumtriphosphat behandelt, zu einem IgG-, IgM- und IgA-haltigen Präparat aufgearbeitet, auf einen Proteingehalt von 5 % eingestellt und mit β-Propiolacton (0,14 %) sterilisiert werden.

Die nach den vorstehenden Verfahren erhaltenen, sterilisierten immunglobulinhaltigen Lösungen werden gewünschtenfalls in bekannter Weise lyophilisiert.

Die so hergestellten immunglobulinhaltigen Präparate wurden zur Herstellung von Arzneimitteln zur therapeutischen Behandlung und Prophylaxe von AIDS beim Menschen verwendet. Gemäß der jeweiligen Formulierung sind sie zur intramuskulären, intravenösen, intrathekalen, intralymphatischen, oralen oder rektalen Anwendung geeignet. Besonders bevorzugt werden Lösungen mit einem Proteingehalt von 5 %, die für die intravenöse Anwendung geeignet sind.

Für die Anwendung derartiger Präparate empfiehlt sich die prophylaktische Gabe bei Risikopersonen. Die postexpositionelle Prophylaxe oder die Gabe zur Therapie bei erkrankten Personen kann erfolgen, um durch zugeführte neutralisierende Antikörper eine HTLV-III-Ausbreitung zu verhindern oder bei einem bereits infizierten Patienten eine Verschlechterung des Krankheitszustandes zu vermeiden.

Um die Wirksamkeit der erfindungsgemäß hergestellten immunglobulinhaltigen Präparate zu zeigen, wurden aus humanem Plasma Immunglobulin-Präparate mit unterschiedlichen Antikörper-Aktivitäten gegen HTLV-III/LAV durch Cohn-Fraktionierung nach der modifzierten Methode von Kistler und Nitschmann mit einem Proteingehalt von 5 % hergestellt. In einem kompetitiven Assay (Tedder, R.S. et al., Lancet 1985 i, 815) zum Nachweis von anti-HTLV-III-Antikörpern wurde geprüft, bis zu welcher Verdünnung die Antikörpertiter gegen HTLV-III/LAV in diesen Präparationen nachweisbar waren. In der nachstehenden Tabelle I ist die Verdünnung der Präparate, die noch eine positive Reaktion zeigten, angegeben.

**TABELLE I**

| Anti-HTLV-III-Aktivität in Immunglobulinpräparaten aus den Cohnfraktionen II und III | | | | |
|---|---|---|---|---|
| Charge | Cohn-Fraktion | reziproke Titer | | reaktive Banden** |
| | | Elisa | IFT* | |
| A | III | 200 | 128 | 24, 41, 55, 76 |
| B | III | 20000 | >512 | 16, 24, 41, 55, 76 |
| C | III | 100 | 32 | 16, 24, 41, 55, 76 |
| D | II | 20000 | >512 | 24, 30, 41, und weitere höhere Banden |
| E | II | 3200 | -- | 18, 24, 32, 59, 65,72 |

| | | | | |
|---|---|---|---|---|
| * Immunfluoreszenztest | | | | |
| ** Westernblot (Molekulargewicht in kilo Dalton) | | | | |

Es ist zu erkennen, daß selbst in mehr als 10000-facher Verdünnung in ausgewählten Immunglobulin-Präparationen noch eine Aktivität nachweisbar ist. Es wird deutlich, daß Antikörper gegen eine Vielzahl von Virusproteinen in diesen Präparaten enthalten sind (Westernblot).

In einem weiteren Versuch zur Wirksamkeit wurden drei Chargen von Immunglobulinen mit Antikörperaktivität gegen HTLV-III nach dem Cohn-Verfahren hergestellt. Die aus der Cohn-Paste-II erhaltenen Immunglobuline wurden mit β-Propiolacton (0,14 %) behandelt, auf 5 % Protein eingestellt und auf Antikörper gegen HTLV-III geprüft. Um die neutralisierende Wirkung dieser Antikörper zu bestimmen, wurden die Gammaglobulinproben verdünnt und mit HTLV-III inkubiert. Bestimmt wurde die Gammaglobulinverdünnung, welche noch eine 50%ige Reduktion des HTLV-III-Infektionstiters verursacht. Der HTLV-III-Infektionstiter vor und nach Inkubation wurde nach dem in Cancer Research (Suppl.) 45 (1985), S. 4592s beschriebenen Infektionstest auf H9 Zellen bestimmt. Das Ergebnis zeigt Tabelle II. Neutralisierende Antikörper wurden bis zu einer Verdünnung von 1:256 nachgewiesen.

**TABELLE II**

| Charge | F | G | H |
|---|---|---|---|
| Verdünnung | | | |
| unverdünnt | ++ | ++ | ++ |
| 1:2 | ++ | ++ | ++ |
| 1:4 | ++ | ++ | ++ |
| 1:8 | ++ | ++ | ++ |
| 1:16 | ++ | ++ | ++ |
| 1:32 | ++ | ++ | ++ |
| 1:64 | ++ | ++ | -- |
| 1:128 | +- | +- | -- |
| 1:256 | +- | -- | -- |
| Ergebnis: | >256/64 | 128/64 | 32/32 |

Um weiterhin die Abwesenheit von Viruspartikeln bzw. von HTLV-III-spezifischen Antigenen in den erfindungsgemäß hergestellten Immunglobulinpräparaten, die eine Voraussetzung für deren Anwendbarkeit ist, zu zeigen, wurde ferner geprüft, ob in anti HTLV-III positiven Immunglobulinpräparationen HTLV-III-Antigene enthalten sind. Das Ergebniss eines Versuchs, bei welchem handelsübliche Mikrotiterplatten (Immunolon) mit fixiertem anti-HTLV-III-positiven Kaninchenserum mit sieben erfindungsgemäß hergestellten Immunglobulinproben inkubiert wurden, zeigt Tabelle III. In keinem Fall wurde mittels Enzym markierter Antikörper (spezifisch für HTLV-III), virusspezifisches Antigen nachgewiesen.

**Nachweis von HTLV-III spezifischen Antigenen in**

| sieben Immunglobulinpräparaten | |
|---|---|
| Charge (unverdünnt) | Antigennachweis |
| I | negativ |
| E | " |
| K | " |
| L | " |
| M | " |
| N | " |
| O | " |

In klinischen Studien wurde überraschend gefunden, daß erfindungsgemäß hergestellte Arzneimittel sehr gut verträglich sind und in Prophylaxe und Therapie von AIDS und insbesondere bei niedrigem T-Zellen-Spiegel oder dessen fortschreitender Abnahme erfolgreich eingesetzt werden können.

Die Erfindung wird durch die folgenden Beispiele erläutert:

### Beispiel 1

Plasmen von Spendern mit Antikörper gegen HTLV-III wurden mittels Enzymimmunoassay (ELISA) ausgewählt und wie folgt aufgearbeitet:
2,3 Liter Plasma (anti-HTLV-III-positiv) wurden mit 2,2 % kolloidaler Kieselsäure (Aerosil^{(R)}) während 3 h bei 45°C behandelt. Der Kieselsäureniederschlag wurde abzentrifugiert und der Überstand durch eine kombinierte Behandlung mit 0,14 % β-Propiolacton und UV-Bestrahlung (1mM/cm².min.) sterilisiert.

Danach wurde der sterilisierte Überstand auf 3,0 % Protein eingestellt und mit 8,4 g Rivanol pro Liter versetzt. Der Niederschlag wurde durch Filtration entfernt, der Überstand mit NaCl versetzt, mittels Zentrifugation und Filtration geklärt und einer Ammonsulfatfällung (310 g/l) unterzogen. Der immunglobulinhaltige Niederschlag wurde durch Zentrifugation gewonnen, in physiologischer NaCl-Lösung gelöst und dialysiert.
Erhalten wurden 220 ml einer 5 % Immunglobulinlösung, welche für die intravenöse Applikation geeignet ist.

### Beispiel 2

Pool-Plasma mit nachgewiesenen Antikörpern gegen HTLV-III (ELISA-Test) wurde mittels Cohnfraktionierung nach Kistler und Nitschmann (Vox Sang. 1962; 7, 414-424) zu Paste II aufgearbeitet. Die Paste wurde in physiologischer NaCl-Lösung gelöst und mit β-Propiolacton (0,14 %) sterilisiert. Auf diese Weise wurde ein Immunglobulin-Präparat gewonnen, welches im competitiven Assay einen Antikörpertiter von 1:100 000 aufwies.

## Patentansprüche

1. Verwendung eines immunglobulinhaltigen Präparates, erhalten durch Aufarbeiten von potentiell infektösem, anti-HTLV-III/LAV-positivem Blut, Plasma, Serum oder deren Derivaten auf eine immunglobulinhaltige Lösung, Sterilisation oder gegebenenfalls Lyophilisation, zur Herstellung eines Arzneimittels zur therapeutischen Behandlung und Prophylaxe von AIDS beim Menschen.

2. Verwendung nach Anspruch 1, dadurch gekennzeichnet, daß das Präparat Immunglobuline der Klassen IgG und/oder IgM und/oder IgA enthält.

3. Verwendung nach einem der Ansprüche 1 oder 2, dadurch gekennzeichnet, daß die Sterilisation durch Behandlung mit β-Propiolacton und/oder durch UV-Strahlen erfolgt.

## Claims

1. Use of an immunoglobulin-containing preparation, obtained by working up potentially infectious anti-HTLV-III/LAV-positive blood, plasma, serum or the derivatives thereof into an immunoglobulin-containing solution, sterilising or optionally lyophilising, in the preparation of a medicament for the therapeutic treatment and prophylaxis of AIDS in humans.

2. Use according to claim 1, characterised in that the preparation contains immunoglobulins of classes IgG and/or IgM and/or IgA.

3. Use according to either of claims 1 and 2, characterised in that the sterilisation is effected by treatment with β-propiolactone and/or by UV rays.

## Revendications

1. Application d'une préparation contenant de l'immunoglobuline, obtenue par traitement de sang, plasma, sérum ou dérivés de ces corps anti-HTLV-III/LAV-positifs potentiellement infectieux afin d'obtenir une solution contenant de l'immunoglobuline, par stérilisation ou éventuellement lyophilisation , dans le but de préparer un médicament de traitement thérapeutique et de prophylaxie du SIDA chez l'homme.

2. Application selon la revendication 1, caractérisée en ce que la préparation contient des immunoglobulines de classes IgG et/ou IgM et/ou IgA.

3. Application selon l'une des revendications 1 ou 2, caractérisée en ce qu'on procède à la stérilisation par traitement avec la β-propiolactone et/ou par irradiation aux UV.
